# FASCICULE DE BREVET EUROPEEN

(11) **EP 0 711 360 B1**
(45) Date de publication et mention de la délivrance du brevet: **11.03.1998**
(21) Numéro de dépôt: 94923761.4
(22) Date de dépôt: 28.07.1994
(51) Int. Cl.: C12Q 1/04, C12Q 1/10

(54) **Procédé d'identification de microorganismes à l'aide d'au moins deux chromogènes**
Verfahren zur Bestimmung von Mikroorganismen mittels mindestens zwei Chromogene
Process for the identification of microorganisms using at least two chromogens

(30) Priorité: 28.07.1993 FR 9309294
(43) Date de publication de la demande: 15.05.1996
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: FR9400958
(87) Numéro de publication internationale: WO9504157

(56) Documents cités:
- EP-A- 0 065 137
- EP-A- 0 451 775
- WO-A-86/05206
- FR-A- 2 684 110
- US-A- 5 210 022

## Description

La présente invention concerne un procédé de mise en évidence de la présence ou de l'absence d'une souche de microorganisme particulière dans un milieu de culture.

La détection de microorganisme est très importante notamment dans l'industrie alimentaire, au niveau du contrôle des eaux ou en médecine, sachant que ces microorganismes peuvent non seulement se révéler être des agents pathogènes, mais également consister en des agents mettant en évidence certains types de contaminations.

Différents procédés permettent de mettre en évidence la présence de microorganismes, dans un milieu quelconque, consistant à prélever un échantillon du milieu considéré, puis à favoriser le développement des microorganismes présents par culture sur ou dans un milieu approprié.

De manière à simplifier la mise en évidence des microorganismes présents, il a été proposé d'utiliser dans le milieu de détection des composés colorés dont la présence est caractéristique d'un microorganisme donné.

La coloration est souvent révélatrice d'une activité enzymatique liée au microorganisme considéré, et le résultat de cette activité peut résulter en une modification du pH du milieu, mise en évidence par un indicateur coloré (EP.A.0 395 532), ou encore en la libération d'un composé chromophore ou fluorophore (FR-A-2 684 110).

Les chromophores ou fluorophores sont des composés généralement obtenus par hydrolyse enzymatique de composés chromogènes ou fluorogènes correspondants, présents dans le milieu de culture.

Les fluorophores émettent un rayonnement caractéristique par fluorescence.

Les composés chromophores sont caractérisés par une couleur d'une longueur d'onde dominante.

Parmi les composés chromophores connus, on notera notamment les dérivés de l'indoxyle, de l'hydroquinoline ou encore les dérivés naphtoïques, ou les dérivés du naphtyle et du phényle.

De manière à différencier deux genres de microorganismes différents dans un milieu de culture, il a même été proposé d'introduire deux agents chromogènes, chacun libérant un composé chromophore d'une couleur caractéristique de la présence d'un microorganisme particulier (US-A-5 210 022).

Bien que l'ensemble de ces milieux soit performant pour la détection de microorganismes d'un genre spécifique, comme par exemple Salmonella, Candida ou E.coli, et leur distinction parmi d'autres espèces, ils ne permettent toutefois pas la détection d'un grand nombre de microorganismes de genres différents sur un même milieu de culture, ou encore parmi les microorganismes d'un même genre la différenciation des espèces pathogènes des autres.

Une telle distinction parait d'autant plus importante pour certaines espèces de levures comme Candida albicans qui est responsable de plus de 50% des pathologies liées aux levures.

Or, on a mis en évidence d'une manière inattendue que l'on pouvait observer au minimum quatre colorations différentes permettant de caractériser les souches en introduisant dans le milieu de culture au moins deux chromogènes substrats d'enzymes d'une souche particulière, les deux chromogènes étant choisis pour que dans le milieu de culture la présence d'au moins une souche soit révélée par une tierce couleur, à savoir :
- les deux couleurs des chromophores correspondant aux chromogènes choisis,
- la couleur correspondant au mélange des chromophores et
- la tierce couleur.

La tierce couleur ne correspond pas au mélange des couleurs des deux chromophores correspondants et est une couleur dont la longueur d'onde dominante ne correspond pas à la longueur d'onde dominante du mélange des chromophores libérés par les chromogènes présents dans le milieu de culture pris isolément.

La longueur d'onde dominante des chromophores et des tierces couleurs pourra être calculée en référence à la lumière du jour, telle que définie par la CIE (Commission Internationale de l'Energie) comme illuminant D6s, à partir de toute méthode standard de mesure de couleur d'objet, en particulier avec un spectrocolorimètre.

La présente invention concerne donc un procédé de mise en évidence de la présence ou de l'absence d'une souche de microorganisme particulière dans un milieu de culture, pour lequel on introduit au moins deux chromogènes substrats d'enzyme de ladite souche, lesdits chromogènes étant choisis pour que dans le milieu de culture la présence de ladite souche soit révélée par une tierce couleur.

Les chromogènes sont notamment substrats des enzymes suivantes: β-galactosidase, β-glucosidase, β-glucuronidase, α-glucosidase, α-galactosidase, phosphatase, N-acetyl-β-glucosidase, N-acetyl-β-galactosidase, α-mannosidase, sulfatase, estérase, lipase et peptidase.

Les chromogènes sont avantageusement choisis parmi les composés d'une même famille chimique, de préférence parmi ceux qui libèrent par hydrolyse deux chromophores différents qui peuvent subir une réaction de couplage.

Par réaction de couplage, on entend toute interaction physicochimique par laquelle la longueur d'onde dominante résultante est différente de la longueur d'onde dominante du mélange des deux chromophores pris isolément.

D'une manière préférentielle, les chromogènes sont de la famille des indoxyles, en particulier des dérivés indoxyle alkylés, halogénés ou dihalogénés.

Parmi les chromophores dérivés de l'indoxyle préférés, on trouve les dérivés indoxyle, bromo-indoxyle, chloro-indoxyle, dichloro-indoxyle, chloro-bromo-indoxyle, tri-chloro-indoxyle et méthyl-indoxyle, en particulier les dérivés suivants: 6-chloro-indoxyle, 5-bromo-indoxyle, 3-bromo-indoxyle, 4,6-dichloro-indoxyle, 6,7-dichloro-indoxyle, 5-bromo-4-chloro-indoxyle, 5-bromo-6-chloro-indoxyle ou 4,6,7-trichloro-indoxyle.

Par microorganisme dont la présence ou l'absence est mise en évidence par le procédé selon l'invention, on entendra les levures, les champignons unicellulaires ou moisissures et les bactéries.

Le procédé selon l'invention est particulièrement adapté pour la mise en évidence de la présence ou l'absence des levures du genre Candida, en particulier Candida albicans et Candida tropicalis.

De même, parmi les bactéries dont la présence ou l'absence est susceptible d'être mise en évidence par le procédé selon l'invention, on trouve notamment les bactéries du genre Streptococcus, Klebsiella, Enterobacter, Escherichia Citrobacter Staphylococcus, Listeria, Clostridium ou Proteus.

Par ailleurs, il a été également constaté que l'adjonction d'un hydrate de carbone dans le milieu de culture à une concentration élevée permettait d'augmenter le nombre de couleurs susceptibles d'être obtenues par le procédé selon l'invention, c'est à dire pour un nombre limité de chromogènes d'obtenir un nombre élevé de couleurs distinctes permettant de distinguer autant de microorganismes différents.

En conséquence, la présente invention concerne un procédé tel que défini précédemment, pour lequel le milieu de culture présente une concentration élevée en hydrate de carbone, de préférence en glucose dans un milieu à base de peptone.

La concentration élevée en hydrate de carbone est avantageusement comprise entre 10 et 30 g/l.

De même, lorsqu'au moins l'un des chromogènes est substrat de la phosphatase, il est avantageux d'employer un milieu comprenant une concentration élevée en phosphate, de préférence comprise entre 1 et 3 g/l.

Les exemples reportés dans les Tableaux ci-après permettent d'illustrer le procédé selon l'invention sans toutefois chercher à en limiter la portée.

**Tableau I:**

| **Exemples de chromophores simples et de tierces couleurs** | |
|---|---|
| Les couleurs suivantes ont été observées pour des chromogènes correspondant à différents chromophores et pour les mélanges dans un même milieu dont la composition est donnée ci-après (g/l): agar (15), peptone (5), NaCl(5), extrait de levure (2) et extrait de viande (1). | |

| Chromophore | Couleur |
|---|---|
| 5-bromo-4-chloro-3-indoxyle | bleuâtre |
| 5-bromo-6-chloro-3-indoxyle | rougeâtre |
| 6-chloro-3-indoxyle | roseâtre |
| méthyl-indoxyle | incolore |
| 5-bromo-4-chloro-3-indoxyle 5-bromo-6-chloro-3-indoxyle | bleu métallique* |
| 5-bromo-4-chloro-3-indoxyle 6-chloro-3-indoxyle | violet métallique* |
| 6-chloro-3-indoxyle méthyl-indoxyle | violacé* |

| | |
|---|---|
| * tierce couleur | |

**Tableau II:**

| **Exemples de coloration selon l'invention pour l'identification directe d' espèces variées de bactéries, distinguant également E.coli des coliformes** | | | |
|---|---|---|---|
| Les milieux ci-après on été préparé dans une base en g/l agar (15), peptone (5), NaCl (5), extrait de levure (2) et extrait de viande (1) avec : A (mg/l) : phenyl glucuronide (100), 5-bromo-4-chloro-3-indoxyl-glucuronide (50) , 5-bromo-6-chloro-3-indoxyl-glucoside (50). B(mg/l): 5-bromo-4-chloro-3-indoxyl-galactoside (50), 5-bromo-6-chloro-3-indoxyl-glucoside (50). C (g/l): 5-bromo-4-chloro-3-indoxyl-glucoside (50), 6-chloro-3-indoxyl-galactoside (50 ). | | | |

| | A | B | C |
|---|---|---|---|
| *Streptococcus D* | *rougeâtre* | *rougeâtre* | *bleu* |
| *Klebsiella* | *rougeâtre* | *bleu-métallique** | *violet-métallique** |
| *Enterobacter* | *rougeâtre* | *bleu-métallique** | *violet-métallique** |
| *Enterobacter MUG+* | *bleu-métallique** | | |
| *E. coli* | *bleu* | *bleu* | *roseâtre* |
| *Citrobacter* | *rougeâtre* | *bleu-métallique** | *violet-métallique** |
| *Proteus mirabilis* | *incolore* | *incolore* | *incolore* |

| | | | |
|---|---|---|---|
| * tierce couleur | | | |

Les couleurs données pour les milieux standard sont les suivantes :

*Streptococcus D* (bleu), *Klebsiella* (bleu), *Enterobacter* (bleu), *Enterobacter MUG+* (bleu), *E. coli* (incolore), *Citrobacter* (incolore).

**Tableau III:**

| **Exemples de coloration selon l'invention pour des espèces variées de levure, mettant en évidence une tierce couleur et un effet de coloration dû à la présence de glucose et de phosphate** | | |
|---|---|---|
| Les milieux ci-après ont été préparés: D(g/l): (comparatif)agar (15), peptone (5), extrait de levure (2), extrait de viande (1),NaCl(5), 5-bromo-4-chloro-3-indoxyl-N-acétyl-glucosaminide (0,1). E (g/l): agar (15), peptone (10), glucose (20), phosphate (2), 5-bromo-4-chloro-3- indoxyl-N-acétyl-glucosaminide (0,1), 5-bromo-6-chloro-3-indoxyl-phosphate (0,1). | | |

| | D | E |
|---|---|---|
| *Candida albicans* | *bleuâtre* | *vert-bleu*** |
| *Candida glabrata* | *incolore* | *rose-violet*** |
| *Candida guilliermondii* | | *rose-violet pâle* ** |
| *Candida krusei* | | *rose-violet*** |
| *Candida lusitaniae* | | *rose-violet pâle* ** |
| *Candida parapsilosis* | | *blanche-grise* ** |
| *Candida tropicalis* | *bleuâtre* | *bleu-métallique* * *(avec halo)* |
| *Cryptococcus neoformans* | | *blanche-rose* ** |
| *Trichosporon beigelii* | | *rose-grise* ** |

| | | |
|---|---|---|
| * tierce couleur, **effet de milieu | | |

Les résultats ci-dessus montrent que l'adjonction de glucose et de phosphate permet d'élargir la gamme de couleurs disponibles pour un même milieu, permettant ici de distinguer sept espèces différentes, et en particulier de repérer Candida albicans sans ambiguité.

## Revendications

1. Procédé de mise en évidence de la présence ou de l'absence d'une souche de microorganismes particulière dans un milieu de culture, caractérisé en ce qu'on introduit dans le milieu de culture au moins deux chromogènes substrats d'enzyme de ladite souche, lesdits chromogènes étant choisis pour que dans le milieu de culture la présence de ladite souche soit révélée par une tierce couleur qui se distingue non seulement de la couleur du chromophore correspondant à chacun des chromogènes mais aussi de la couleur correspondant au mélange des différents chromophores.

2. Procédé selon la revendication 1, caractérisé en ce que les chromogènes sont des composés de la même famille chimique.

3. Procédé selon l'une des revendications 1 et 2, caractérisé en ce que les deux chromogènes libèrent par hydrolyse deux chromophores différents qui peuvent subir une réaction de couplage.

4. Procédé selon l'une des revendications 1 à 3, caractérisé en ce que les chromogènes sont de la famille des indoxyles, en particulier des dérivés indoxyle alkylés, halogénés ou dihalogenés.

5. Procédé selon l'une des revendications 1 à 4, caractérisé en ce que le chromophore est choisi parmi les dérivés indoxyle, bromo-indoxyle, chloro-indoxyle,dichloro-indoxyle,chloro-bromo-indoxyle,tri-chlore-indoxyle et méthylindoxyle, en particulier les dérivés suivants: 6-chloro-indoxyle, 5-bromo-indoxyle, 3-bromo-indoxyle, 4,6-dichloro-indoxyle, 6,7-dichloro-indoxyle, 5-bromo-4-chloro-indoxyle, 5-bromo-6-chloro-indoxyle ou 4,6,7-trichloro-indoxyle.

6. Procédé selon l'une des revendications 1 à 5, caractérisé en ce que les microorganismes sont des levures.

7. Procédé selon la revendication 6, caractérisé en ce que les levures sont du genre Candida.

8. Procédé selon l'une des revendications 1 à 5, caractérisé en ce que les microorganismes sont des bactéries.

9. Procédé selon la revendication 8, caractérisé en ce que les bactéries sont du genre Streptococcus, Klebsiella, Enterobacter, Escherichia, Citrobacter, Staphvlococcus, Listeria, Clostridium ou Proteus.

10. Procédé selon l'une des revendications 1 à 9, caractérisé en ce que le milieu de culture présente une concentration élevée en hydrate de carbone.

11. Procédé selon la revendication 10, caractérisé en ce que le milieu de culture présente une concentration élevée en glucose dans un milieu à base de peptone.

12. Procédé selon l'une des revendications 10 ou 11, caractérisé en ce que la concentration en hydrate de carbone est comprise entre 10 et 30 g/l.

13. Procédé selon l'une des revendications 1 à 12, caractérisé en ce que le chromogène est un substrat de la phosphatase.

14. Procédé selon la revendication 13, caractérisé en ce que le milieu comprend une concentration élevée en phosphate.

15. Procédé selon la revendication 14, caractérisé en ce que la concentration en phosphate est comprise entre 1 et 3 g/l.

## Patentansprüche

1. Verfahren zum Nachweis der Anwesenheit oder Abwesenheit eines speziellen Mikroorganismen-Stammes in einem Kulturmedium, dadurch gekennzeichnet, daß man in das Kulturmedium mindestens zwei Enzym-Substrat-Chromogene des genannten Stammes einführt, wobei die genannten Chromogene so ausgewählt werden, daß in dem Kulturmedium die Anwesenheit des genannten Stammes durch eine dritte Farbe angezeigt wird, die sich nicht nur von der Farbe des jedem der Chromogene entsprechenden Chromophors, sondern auch von der der Mischung der verschiedenen Chromophore entsprechenden Farbe unterscheidet.

2. Verfahren nach Anspruch 1, dadurch gekennzeichnet, daß es sich bei den Chromogenen um Verbindungen der gleichen chemischen Familie handelt.

3. Verfahren nach einem der Ansprüche 1 und 2, dadurch gekennzeichnet, daß die beiden Chromogene durch Hydrolyse zwei verschiedene Chromophore freisetzen, die eine Kupplungs-Reaktion eingehen können.

4. Verfahren nach einem der Ansprüche 1 bis 3, dadurch gekennzeichnet, daß die Chromogene solche aus der Familie der Indoxyle, insbesondere der alkylierten, halogenierten oder dihalogenierten Indoxyl-Derivate sind.

5. Verfahren nach einem der Ansprüche 1 bis 4, dadurch gekennzeichnet, daß der Chromophor ausgewählt wird unter den Indoxyl-, Bromoindoxyl-, Chloroindoxyl-, Dichloroindoxyl-, Chlorobromoindoxyl-, Trichloroindoxyl- und Methylindoxyl-Derivaten, insbesondere den 6-Chloroindoxyl-, 5-Bromoindoxyl-, 3-Bromoindoxyl-, 4,6-Dichloroindoxyl-, 6,7-Dichloroindoxyl-, 5-Bromo-4-chloroindoxyl-, 5-Bromo-6-chloroindoxyl- oder 4,6,7-Trichloroindoxyl-Derivaten.

6. Verfahren nach einem der Ansprüche 1 bis 5, dadurch gekennzeichnet, daß es sich bei den Mikroorganismen um Hefen handelt.

7. Verfahren nach Anspruch 6, dadurch gekennzeichnet, daß es sich bei den Hefen um solche des Genus Candida handelt.

8. Verfahren nach einem der Ansprüche 1 bis 5, dadurch gekennzeichnet, daß es sich bei den Mikroorganismen um Bakterien handelt.

9. Verfahren nach Anspruch 8, dadurch gekennzeichnet, daß es sich bei den Bakterien um solche des Genus Streptococcus, Klebsiella, Enterobacter, Escherichia, Citrobacter, Staphylococcus, Listeria, Clostridium oder Proteus handelt.

10. Verfahren nach einem der Ansprüche 1 bis 9, dadurch gekennzeichnet, daß das Kulturmedium eine erhöhte Konzentration an Kohlenhydrat enthält.

11. Verfahren nach Anspruch 10, dadurch gekennzeichnet, daß das Kulturmedium eine erhöhte Konzentration an Glucose in einem Medium auf Pepton-Basis enthält.

12. Verfahren nach einem der Ansprüche 10 oder 11, dadurch gekennzeichnet, daß die Kohlenhydrat-Konzentration zwischen 10 und 30 g/l liegt.

13. Verfahren nach einem der Ansprüche 1 bis 12, dadurch gekennzeichnet, daß es sich bei dem Chromogen um ein Substrat der Phosphatase handelt.

14. Verfahren nach Anspruch 13, dadurch gekennzeichnet, daß das Medium eine erhöhte Konzentration an Phosphat enthält.

15. Verfahren nach Anspruch 14, dadurch gekennzeichnet, daß die Phosphat-Konzentration zwischen 1 und 3 g/l liegt.

## Claims

1. Method for demonstrating the presence or absence of a particular strain of microorganism in a culture medium, characterized in that at least two chromogens which are enzyme substrates for said strain are introduced into the culture medium, said chromogens being chosen so that, in the culture medium, the presence of said strain is revealed by an accessory colour which is distinct, not only from the colour of the chromophore corresponding to each of the chromagens, but also from the colour corresponding to the mixture of the different chromophores.

2. Method according to Claim 1, characterized in that the chromogens are compounds of the same chemical family.

3. Method according to either of Claims 1 and 2, characterized in that the two chromogens liberate on hydrolysis two different chromophores which can undergo a coupling reaction.

4. Method according to one of Claims 1 to 3, characterized in that the chromogens are of the indoxyl family, especially alkylated, halogenated or dihalogenated indoxyl derivatives.

5. Method according to one of Claims 1 to 4, characterized in that the chromophore is chosen from the indoxyl derivatives bromoindoxyl, chloroindoxyl, dichloroindoxyl, chlorobromoindoxyl, trichloroindoxyl and methylindoxyl, and especially the following derivatives: 6-chloroindoxyl, 5-bromoindoxyl, 3-bromoindoxyl, 4,6-dichloroindoxyl, 6,7-dichloroindoxyl, 5-bromo-4-chloroindoxyl, 5-bromo-6-chloroindoxyl or 4,6,7-trichloroindoxyl.

6. Method according to one of Claims 1 to 5, characterized in that the microorganisms are yeasts.

7. Method according to Claim 6, characterized in that the yeasts are of the genus Candida.

8. Method according to one of Claims 1 to 5, characterized in that the microorganisms are bacteria.

9. Method according to Claim 8, characterized in that the bacteria are of the genus Streptococcus, Klebsiella, Enterobacter, Escherichia, Citrobacter, Staphylococcus, Listeria, Clostridium or Proteus.

10. Method according to one of Claims 1 to 9, characterized in that the culture medium possesses a high carbohydrate concentration.

11. Method according to Claim 10, characterized in that the culture medium possesses a high glucose concentration in a peptone-based medium.

12. Method according to either of Claims 10 and 11, characterized in that the carbohydrate concentration is between 10 and 30 g/l.

13. Method according to one of Claims 1 to 12, characterized in that the chromogen is a substrate for phosphatase.

14. Method according to Claim 13, characterized in that the medium comprises a high phosphate concentration.

15. Method according to Claim 14, characterized in that the phosphate concentration is between 1 and 3 g/l.
